# EUROPEAN PATENT APPLICATION

(11) **EP 2 371 379 A2**
(43) Date of publication of application: **05.10.2011**
(21) Application number: 11162700.6
(22) Date of filing: 26.06.2007
(51) Int. Cl.: A61K 38/18, A61K 47/36, A61K 31/727, A61K 31/737, A61K 31/717, A61P 1/18, A61P 3/10, A61P 25/00

(54) **New formulation for increasing bioavailability of neurturin**

(30) Priority: 26.06.2006 EP 06013135
(62) Divisional of application: 07764869.9
(71) Applicant: DeveloGen Aktiengesellschaft, 37079 Göttingen (DE)
(72) Inventor: Harder, Friedrich, 88131, Lindau (DE); Austen, Matthias, 37079, Göttingen (DE)
(74) Representative: Couchman, Jonathan Hugh

(57) **Abstract**

The present invention relates to formulations with protein growth factors, particularly neurturin as active ingredients and low molecular weight polyanionic excipients having increased bioavailability.

## Description

### Field of the Invention

The present invention relates to formulations with protein growth factors, particularly neurturin as active ingredients and low molecular weight polyanionic excipients having increased bioavailability.

### Background

Pancreatic beta-cells secrete insulin in response to elevated blood glucose levels. Insulin amongst other hormones plays a key role in the regulation of the fuel metabolism. Insulin leads to the storage of glycogen and triglycerides and to the synthesis of proteins. The entry of glucose into muscles and adipose cells is stimulated by insulin. In patients who suffer from diabetes mellitus type I or LADA (latent autoimmune diabetes in adults, Pozzilli & Di Mario, 2001, Diabetes Care. 8:1460-1467) beta-cells are being destroyed due to autoimmune attack. The amount of insulin produced by the remaining pancreatic islet cells is too low, resulting in elevated blood glucose levels (hyperglycemia). In diabetes mellitus type II liver and muscle cells loose their ability to respond to normal blood insulin levels (insulin resistance). High blood glucose levels (and also high blood lipid levels) in turn lead to an impairment of beta-cell function and to an increase in beta-cell death. Interestingly the rate of beta-cell neogenesis and replication does not appear to increase in type II diabetics, thus causing a reduction in total beta-cell mass over time. Eventually the application of exogenous insulin becomes necessary in type II diabetics.

In type I diabetics, where beta-cells are being destroyed by autoimmune attack, treatments have been devised which modulate the immune system and may be able to stop or strongly reduce islet destruction (Raz et al., 2001, Lancet 358:1749-1753; Chatenoud et al., 2003, Nat Rev Immunol. 3: 123-132; Homann et al., Immunity. 2002, 3:403-415). However, due to the relatively slow regeneration of human beta-cells such treatments can be more successful if they are combined with treatments that can stimulate beta-cell regeneration.

Diabetes is a very disabling disease, because today's common anti-diabetic drugs do not control blood sugar levels well enough to completely prevent the occurrence of high and low blood sugar levels. Frequently elevated blood sugar levels are toxic and cause long-term complications like for example nephropathy, retinopathy, neuropathy and peripheral vascular disease, Extensive loss of beta cells also leads to deregulation of glucagon secretion from pancreatic alpha cells which contributes to an increased risk of dangerous hypoglycemic episodes. There is also a host of related conditions, such as obesity, hypertension, heart disease and hyperlipidemia, for which persons with diabetes are substantially at risk.

Apart from the impaired quality of life for the patients, the treatment of diabetes and its long term complications presents an enormous financial burden to our healthcare systems with rising tendency. Thus, for the treatment of diabetes mellitus type I and LADA, but also for the treatment of late stages of diabetes mellitus type II there is a strong need in the art to identify factors that induce regeneration of pancreatic insulin producing beta-cells. These factors could restore normal function of the endocrine pancreas once its function is impaired or event could prevent the development or progression of diabetes type I, LADA or diabetes type II.

Neurturin is expressed in embryonic pancreas, and recombinant neurturin has been shown to stimulate the differentiation of mouse ES-cells into insulin producing cells. Moreover, transgenic mice with elevated neurturin levels in the pancreas have a substantially increased pancreatic beta-cell mass. Based on these findings, the use of neurturin for the treatment of pancreatic disorders such as diabetes has been proposed (see for example WO03/099318 and WO2005/051415 the disclosure of which is herein incorporated by reference).

Neurturin had previously been proposed as a treatment for neurodegenerative diseases such as Parkinsons, Alzheimers and Huntington's disease, motor neuron disorders, spinal cord injuries or hearing disorders (WO 97/08196, WO 99/06064; Akerud et al. J Neurochem. 1999;73(1):70-78; Koeberle & Ball Neuroscience. 2002; 110(3):555-567; Bilak et al. Mol Cell Neurosci. 1999;13(5):326-336; Perez-Navarro et al. Neuroscience. 2000; 98(1):89-96; Rosenblad et al, Eur J Neurosci. 1999; 11 (5):1554-1566, the disclosures of which are herein incorporated by reference).

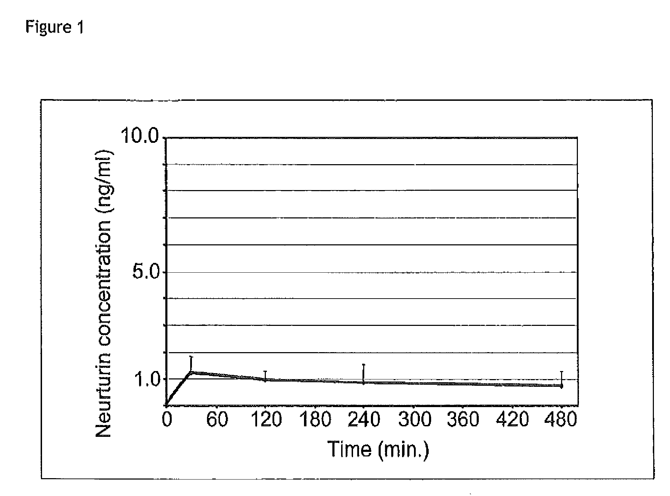

It was found, however, that upon convection-enhanced delivery (CED) into rat brains, the distribution volume of neurturin and the related factor GDNF was limited (Hamilton et al., Exp Neurol. 2001; 168(1):155-161). In our own studies, we found that the bioavailability after subcutaneous and intravenous injection of neurturin Is low, only about 10 percent of subcutaneously injected neurturin enter the circulation (see Fig. 1). It would, therefore, be highly desirable to provide a formulation form that enhance the bioavailability of neurturin.

Heparin has been used extensively in medicine due to Its anticoagulant activities. Inhibition of blood clotting by heparin occurs by binding and inhibition of the factor Xa protease and other serine proteases participating in the blood clotting cascade, and a minimal pentasaccharide required for factor Xa inhibition has been described (Wang et al., J Clin Invest. 2002; 110:127-136; Esko & Lindahl J Clin Invest. 2001;108(2):169-173; Weitz, JL, N Engl J Med. 1997; 337(10):688-698)).

It is known that protein growth factors such as VEGF, bFGF, neurturin and the related protein GDNF bind to heparin (Lin et al J Neurochem. 1994; 63 (2):758-768; WO 97/08196; Kotzbauer et al. Nature. 1996; 384(6608):467-470; Hamilton et al., supra). Unfractionated Heparin consists of glycosaminoglycan polymer chains. These chains contain alternating residues of D-glucosamine and uronic acid (either glucuronic acid or iduronic acid), and have molecular weights up to about 30000 Dalton (Weitz et al., supra).

Hamilton et al. have observed a significantly increased distribution volume upon CED of GDNF and neurturin together with heparin.

For GDNF it has been demonstrated that heparin can enhance binding to its receptor GFRa1, and that heparin-bound GDNF is protected from proteolytic attack (Rickard et al, Glycobiology. 2003; 13(6):419-426).

US 5,849,689 discloses administration of hepatocyte growth factor (HGF) in combination with heparin, low molecular weight heparin and pentosan polysulphate. It was shown that plasma half-life of HGF was significantly enhanced by co-administering HGF and heparin, while coadministration of HGF with low molecular weight heparin led to only small increase in plasma half-life compared to administration of HGF alone.

It was found by the present inventors that the bioavailability of neurturin is, however, only moderately increased by heparin, particularly after subcutaneous administration. Thus, the object of the present invention was to provide novel formulations of protein growth factors, particularly novel formulations of neurturin, which have significantly increased bioavailability.

### Summary of the Invention

The present invention relates to a pharmaceutical formulation comprising a protein growth factor such as neurturin as an active ingredient and a low molecular weight polyanionic polymer together with pharmaceutically acceptable carriers, diluents and/or adjuvants.

In a further embodiment, the present invention relates to a method for administering a protein growth factor to a subject in need thereof, wherein a pharmaceutical formulation is administered comprising a protein growth factor as an active ingredient and a low molecular weight polyanionic polymer together with pharmaceutically acceptable carriers, diluents and/or adjuvants.

In a preferred embodiment of the invention, the protein growth factor is a neurturin protein product.

In another preferred embodiment of the invention, the polyanionic polymer is a low molecular weight heparin or a pentosan polysulphate (PPS).

The formulations of the present invention are particularly suitable for preventing or treating pancreatic disorders, more particularly pancreatic autoimmune disorders, e.g. autoimmune diabetes such as type I diabetes or LADA but also type II diabetes.

The formulations of the present invention are particularly suitable for preventing or treating neurodegenerative disorders, more particularly diabetic polyneuropathy.

### Detailed Description of the Invention

The formulation of the present invention includes a protein growth factor such as neurturin and a polyanionic polymer with low molecular weight.

The protein growth factor is preferably selected from heparin-binding protein growth factors such as neurturin, artemin, persephin, VEGF, bFGF, or GDNF, or pharmaceutically active fragments and derivatives thereof. Especially preferred are human growth factors.

More preferably the protein growth factor is a neurturin protein product, most preferably human neurturin or a pharmaceutically active fragment thereof. Neurturin protein products are preferably produced via recombinant techniques because such methods are capable of achieving high amounts of protein at a great purity, but are not limited to products expressed in bacterial, plant, mammalian, or insect cell systems.

Recombinant neurturin protein product forms include glycosylated and non-glycosylated forms of the protein. In general, recombinant techniques involve isolating the genes encoding for neurturin protein product, cloning the gene in suitable vectors and/or cell types, modifying the gene if necessary to encode a desired variant, and expressing the gene in order to produce the neurturin protein product.

Alternatively, a nucleotide sequence encoding the desired neurturin product may be chemically synthesized. It is contemplated that a neurturin product may be expressed using nucleotide sequences that vary in codon usage due to the degeneration of the genetic code or allelic variations or alterations made to facilitate production of the protein product by the selected cell.

Kotzbauer et al., Nature 384:467-470, describe the identification of a mouse cDNA and amino acid sequence and a human cDNA and amino acid sequence for neurturin protein. The neurturin products according to this invention may be isolated or generated by a variety of means. Exemplary methods for producing neurturin products useful are described in patent application WO 97108196, the disclosures of which are hereby incorporated by reference. Also described are a variety of vectors, host cells, and culture growth conditions for the expression of neuturin protein, as well as methods to synthesize variants of neurturin protein product. Additional vectors suitable for the expression of neurturin protein product in E. coli are disclosed in Patent No. EP 0 423 980, the disclosure of which is hereby incorporated by reference.

The molecular weight of purified neurturin indicates that in its biologically active form the protein is a disulfide-bonded dimer. The material isolated after expression in a bacterial system is essentially biologically inactive, and exists as a monomer. Refolding is necessary to produce the biologically active disulfide-bonded dimer. Processes suitable for the refolding and maturation of the neurturin expressed in bacterial systems are substantially similar to those described in WO93/06116. Standard in vitro assays for the determination of neurturin activity are also substantially similar to those determining GDNF activity as described in W093/06116 and in U.S. Application Serial No. 08/535,681, and are hereby incorporated by reference.

A preferred assay for the determination of neurturin activity is based on binding of neurturin to GDNF family receptor GFRa2 and receptor tyrosine kinase cRet. Thereby MAPK pathway is activated.

The assay uses human neuroblastoma cell lines TGW (JCRB0618) that express GFRa2 and cRet and that are transfected with a luciferase gene under control of repetitive serum response elements (SRE).

Since luciferase expression correlates with MAPK pathway activation, neurturin activity can be determined via expression of luciferase.

Neurturin product variants are prepared by introducing appropriate nucleotide changes into the DNA encoding the polypeptide or by in vitro chemical synthesis of the desired polypeptide. It will be appreciated by those skilled in the art that many combinations of deletions, insertions, and substitutions can be made resulting in a protein product variant presenting neurturin biological activity.

Mutagenesis techniques for the replacement, insertion or deletion of one or more selected amino acid residues are well known to one skilled in the art (e.g., U.S. Patent No. 4,518,584, the disclosure of which is hereby incorporated by reference.)

Neurturin substitution variants have at least one amino acid residue of the human or mouse neurturin amino acid sequence removed and a different residue inserted in its place. Such substitution variants include allelic variants, which are characterized by naturally occurring nucleotide sequence changes in the species population that may or may not result in an amino acid change.

It is preferred that the sequence identity to mature human neurturin is of at least 90%, in particular the identity is of at least 94%, preferably of more than 96%, and still more preferably the sequence identity is of more than 98%.

Chemically modified derivatives of neurturin protein products also may be prepared by one of skill in the art given the disclosures herein. The chemical moieties most suitable for derivatization include water soluble polymers. A water soluble polymer is desirable because the protein to which it is attached does not precipitate in an aqueous environment, such as a physiological environment. Preferably, the polymer will be pharmaceutically acceptable for the preparation of a therapeutic product or composition. One skilled in the art will be able to select the desired polymer based on such considerations as whether the polymer/protein conjugate will be used therapeutically, and if so, the desired dosage, circulation time, resistance to proteolysis, and other considerations. A particularly preferred water-soluble polymer for use herein is polyethylene glycol. Attachment at residues important for receptor binding should be avoided if receptor binding is desired. One may specifically desire an N-terminal chemically modified protein.

The present invention contemplates use of neurturin protein products, e.g. derivatives which are prokaryote-expressed neurturin, or variants thereof, linked to at least one polyethylene glycol molecule, as well as use of neurturin, or variants thereof, attached to one or more polyethylene glycol molecules via an acyl or alkyl linkage. Pegylation may be carried out by any of the pegylation reactions known in the art. See, for example: Focus on Growth Factors, 3 (2):4-10, 1992; EP 0 154 316, the disclosure of which is hereby incorporated by reference; EP 0 401 384; and the other publications cited herein that relate to pegylation.

The present invention also discloses use of derivatives which are prokaryote-expressed neurturin, or variants thereof, linked to at least one hydrophobic residue, for example fatty acid molecule, as well as use of neurturin, or variants thereof, attached to one or more hydrophobic residues. For example, patent application published as WO 03/010185, which is hereby incorporated by reference, describes a method for producing acylated polypeptides in transformed host cells by expressing a precursor molecule of the desired polypeptide which are then to be acylated in a subsequent in vitro step.

The low molecular weight polyanionic polymer may be a synthetic polymer, a naturally occurring polymer or a polymer derived from a naturally occurring polymer by modification and/or chemical or enzymatic fragmentation. The polyanionic polymer contains a plurality of anionic groups such as carboxylate and/or sulphate groups. More preferably, the polyanionic polymer is a sulphate group containing polymer. Preferably, the weight average molecular weight (M_{w}) of the polyanionic polymer up to about 12000 Da and more preferably up to about 8000 Da. Further, it is preferred that the weight average molecular weight (M_{w}) is at least about 200 Da and more preferably at least about 500 Da.

For example, the polyanionic polymer may be selected from low molecular weight sulphated saccharides, sulphated cyclodextrins, or sulphated synthetic polymers such as acrylic polymers, aromatic polymers, and/or polyalcohols. More particularly, the polyanionic polymer is selected from low molecular weight heparins or heparin derivatives, heparan sulphates, chondroitin sulfates, dextran sulphates, pentosan polysulphates or derivatives or combinations thereof.

In the following, non-limiting examples of low molecular weight polyanionic polymers which are suitable for incorporating into the formulation of the present invention are provided.

It should be noted that the content of the following documents is herein incorporated by reference.

Chemically modified heparin-derived oligosaccharides (list from Wang et al. (2002), supra), heparin-like oligosaccharides, dextran sulphates, sulphated low molecular weight glycosaminoglycans, dextrin-2-sulphates, cellulose sulphates and naphthalene sulfonate polymer (e.g. PRO 2000), PAVAS (a co-polymer of acrylic acid with vinyl alcohol sulphate), the sulphonated polymer PAMPS [poly(2-acryl-amido-2-methyl-1-propanesulfonic acid] (M_{w} e.g. approximately 7000-12000), Chondroitin sulphates, Sulphated cyclodextrins, Laminarin sulphate (Alban, S. in Carbohydrates in Drug Design (Ed. Z.J. Witczak, K.A. Nieforth) Dekker, New York, 1997, pp 209.), Polyglycerin sulphates (Türk, H., Haag, R., Alban, S. Bioconjugate Chem. 2004, 15, 162;), Pentosan polysulphates (PPS) and derivatives thereof such lactose-modified pentosan polysulphates, fractionated PPS/low molecular weight PPS, or Fucoidan.

Low molecular weight heparin (LMWH) analogues such as Enoxaparin, Dalteparin, Fragmin, Nadroparin, Tinzaparin, Fondaparinux, Bemiparin, Reviparin, Ardeparin, Certoparin, and/or Parnaparin, e.g. Lovenox^{®}, Fraxiparin^{®}, Sandoparin^{®} or Arixtra^{®}, are preferred examples of suitable polymers. They are obtained by fractionation and/or limited enzymatic or chemical digestion of heparin, and have an average molecular weight of preferably about 3000 to about 7000 Dalton (Weitz 1997 supra).

In treatment schemes for diseases or conditions in which the pharmaceutical regulation of coagulation is not required, anticoagulant activity of an additive or excipient might be an undesired feature. Thus, for the purpose of enhancement of neurturin bioavailability, it is desirable for some embodiments of the invention to use polyanionic polymers which are largely or completely devoid of anticoagulant activities.

Heparin has an antiinflammatory effect due to its ability to inhibit leukocyte adhesion (Wang et al., 2002, supra) and possibly due to its binding to certain cytokines (Kuschert et al. Biochemistry. 1999;38(39):12959-12968.). Modified Heparin derivatives can be made which retain some of the antiinflammatory activity while being devoid of anticoagulant activity or which lack both activities (Wang et al., 2002, supra). Thus, it may be advantageous to combine an anti-diabetic/beta-cell regenerative agent such as neurturin with such an anti-inflammatory heparin derivative, since both type I and type II diabetes have been linked to inflammatory processes (Eisenbarth Adv Exp Med Biol. 2004;552:306-310; Donath et al. Diabetes. 2005;54 Suppl 2:S108-13).

Alternatively, it could be advantageous to combine neurturin with a non-antiinflammatory and non-anticoagulant heparin-like compound to obtain a pharmaceutically neutral additive with only distribution-enhancing properties.

A preferred example for a heparin-related compound with distinct pharmacological properties is pentosan polysulfate (PPS). PPS is a semisynthetic anionic polymer preferably derived from plant material. It is structurally similar to heparin with a molecular weight of preferably about 4000 to about 6000 Dalton. Compared to heparin, it has significantly lower anticoagulant activity, and it is an approved drug for the treatment of inflammatory diseases of the bladder epithelium. It is also used as an antiinflammatory drug for arthritis treatment in animals.

The pharmaceutical formulation of the present invention has an increased bioavailability of the active ingredient compared to a pharmaceutical formulation which does not contain the polyanionic polymer. Preferably, the polyanionic polymer is present In an amount to provide an at least 2-fold, preferably at least 5-fold and more preferably at least 10-fold increase in the bioavailability of the active ingredient.

The increase of bioavailability may be determined as shown in the Examples of the present application. More particularly, a. formulation containing the active ingredient in a given dose and the polyanionic polymer is compared to a pharmaceutical formulation containing the active ingredient in the same dose but without the polyanionic polymer: The bioavailability of both formulations may be determined from the plasma concentration after subcutaneous administration into experimental animals, e.g. mice. Preferably, the plasma-concentration is measured over a time period of 240 min, more preferably of 24h.

The pharmaceutical formulation of the present invention may be adapted for administration by any effective route, e.g. by oral, nasal, rectal, pulmonal, topical, transdermal or parenteral routes of administration. Thus, the formulation may be a solid or liquid formulation, e.g. a tablet, capsule, powder, cream, gel, ointment, solution, emulsion, suspension, lyophilisate etc. Preferably, however, the formulation is administered by injection or infusion, more preferably by injection, e.g. by subcutaneous or intravenous injection. Thus, the pharmaceutical formulation is preferably an aqueous solution.

In addition to the active ingredient and the polyanionic polymer, the pharmaceutical formulation may comprise any other pharmaceutically acceptable carriers, diluents and/or adjuvants, such as buffers, agents for adjusting tonicity, stabilizers, fillers, disintegrants, thickeners, etc.

The pharmaceutical formulation contains the active ingredient in a therapeutically effective dose. The therapeutically effective dose depends on the type of the active ingredient, the type and the variety of the disease to be treated and the type of administration. For parenteral formulations containing neurturin as an active Ingredient, the therapeutically effective dose is preferably in the range of about 0.001 mg to 500 mg, more preferably from about 0.05 to about 100 mg, most preferably from about 0.01 to about 5 mg per day.

The formulation is preferably administered to a mammal, particularly a human, Thus, the formulation is suitable for human and veterinary medicine. The formulation is particularly suitable for the prevention and/or treatment of neurodegenerative or pancreatic disorders, particularly pancreatic autoimmune disorders such as diabetes type I and LADA, or diabetes type II.

The neurturin product may be administered by any suitable means, preferably enterally or parenterally or topically directly to the pancreas, as known to those skilled in the art. The specific dose may be calculated according to considerations of body weight, body surface area or organ size. Further refinement of the calculations necessary to determine the appropriate dosage for treatment involving each of the above mentioned formulations is routinely made by those of ordinary skill in the art and is within the ambit of tasks routinely performed. Appropriate dosages may be ascertained through use of the established assays for determining dosages utilized in conjunction with appropriate dose-response data. The final dosage regimen involved in a method for treating the above described conditions will be determined by the attending physician, considering various factors which modify the action of drugs, e.g., the age, condition, body weight, sex and diet of the patient, the severity of any infection, time of administration and other clinical factors. As studies are conducted, further information will emerge regarding the appropriate dosage levels for the treatment of various diseases and conditions.

It is envisioned that the continuous administration or sustained delivery of a neurturin product may be advantageous for a given treatment. While continuous administration may be accomplished via a mechanical means, such as with an infusion pump, it is contemplated that other modes of continuous or near continuous administration may be practiced. For example, chemical derivatization or encapsulation may result in sustained release forms of the protein having the effect of continuous presence, in predictable amounts, based on a determined dosage regimen. Thus, neurturin protein products include proteins derivatized or otherwise formulated to effectuate such continuous administration.

In a further preferred embodiment, neurturin protein product can be delivered directly to progenitor, e.g. stem cells in order to stimulate the differentiation of insulin producing cells *in vitro* or *in vivo.* In this embodiment of the invention, neurturin may be added preferably to concentrations between 0.1 ng/ml and 500 ng/ml, more preferably between 1 and 100 ng/ml, most preferably 50 ng/ml.

The neurturin protein may be administered either as a monotherapy or as a combination therapy with other pharmaceutical agents. For example, they may be administered together with other pharmaceutical agents suitable for the treatment or prevention of pancreatic diseases and/or obesity and/or metabolic syndrome, particularly with other pharmaceutical agents suitable for stimulating and/or inducing the differentiation of insulin producing cells from progenitor cells. Further, they may be administered together with pharmaceutical agents which have an immunosuppressive activity, e.g. antibodies, polypeptides and/or peptidic or non-peptidic low molecular weight substances as disclosed in WO 2005/051415.

**The figures illustrate the invention:**
**Fig. 1****.** shows plasma neurturin concentration following subcutaneous application of 0.05mg/kg neurturin formulated in 0.9% NaCl solution. Compared to the total injected amount of neurturin, the bioavailability is very low at about 5%.

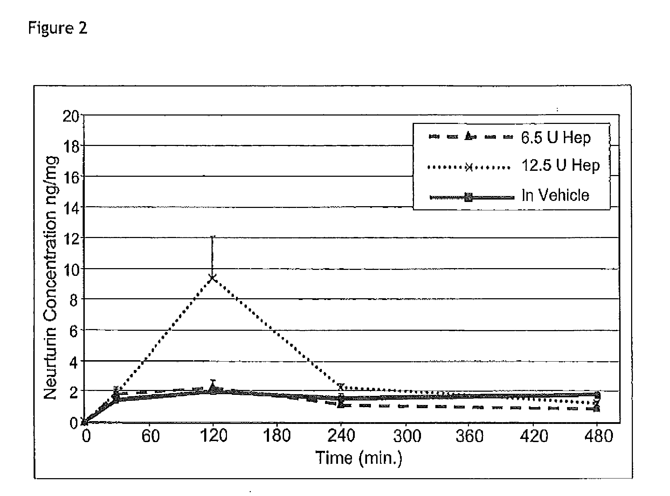

**Fig. 2** shows plasma neurturin concentration following subcutaneous application of 0.25mg/kg neurturin formulated in 0.9% NaCl solution alone or together with Heparin. The figure shows that compared to the bioavailability of neurturin formulated in physiological saline the bioavailability of neurturin/12.5 U heparin formulation is only twofold increased.

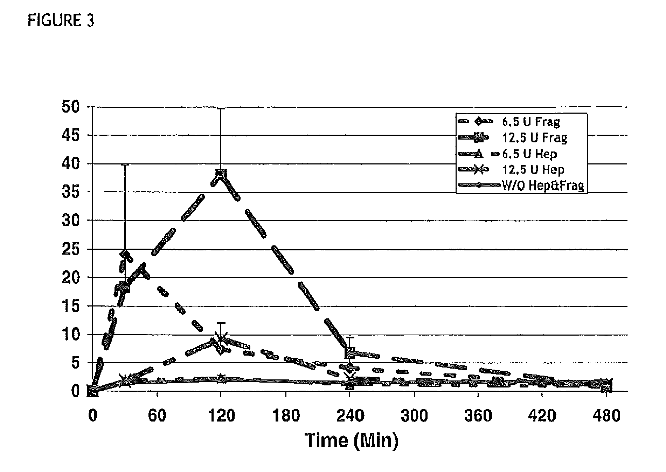

**Fig. 3** shows plasma neurturin concentration following subcutaneous application of 0.25mg/kg neurturin formulated in 0.9% NaCl solution alone or together with Heparin or a low molecular weight heparin (LMWH). Compared to the bioavailability of neurturin formulated in physiological saline the bioavailability of a neurturin formulation containing 12.5 units of a LMWH as an excipient is significantly about tenfold increased.

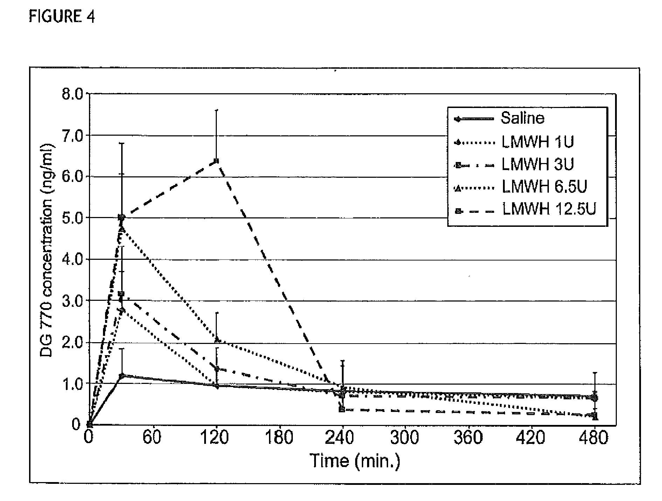

**Fig. 4** shows plasma neurturin concentration following subcutaneous application of 0.05mg/kg neurturin formulated in 0.9% NaCl solution alone or together with a low molecular weight heparin (LMWH). Addition of a LMWH to the injected neurturin dose dependently increases plasma concentration of neurturin.

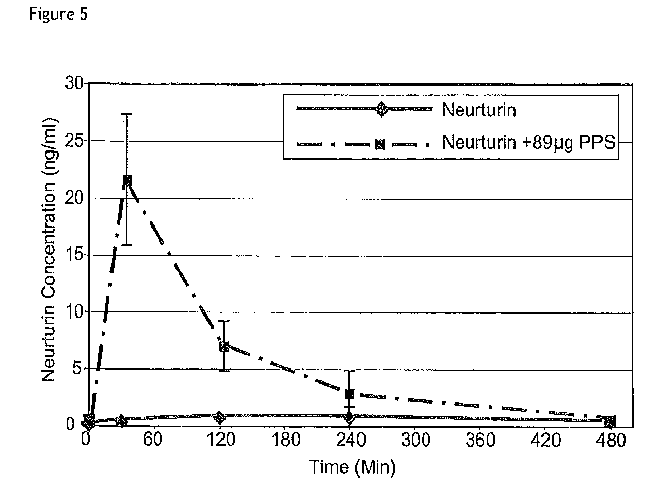

**Fig. 5** shows plasma neurturin concentration following subcutaneous application of 0.05mg/kg neurturin formulated in 0.9% NaCl solution alone or together with a pentosan polysulfat (PPS). PPS compared to heparin or a LMWH increases neurturin plasma concentrations more efficiently when given at the same molar ratio.

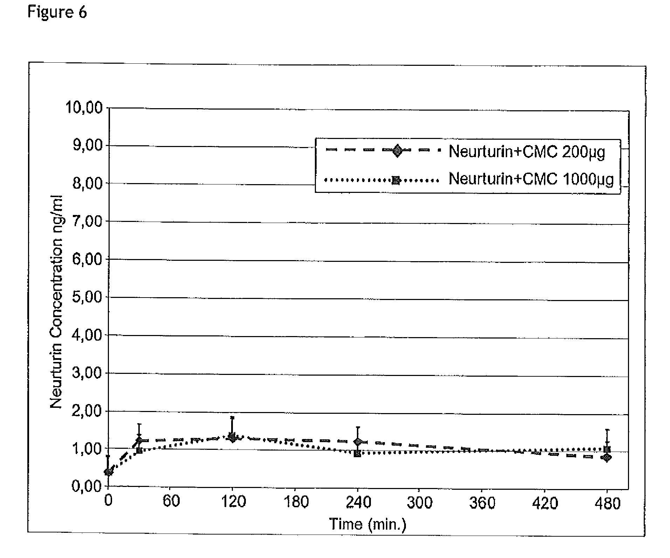

**Fig. 6** shows plasma neurturin concentration following subcutaneous application of 0.05mg/kg neurturin formulated in 0.9% NaCl solution together with carboxymethylcellulose (CMC). Addition of CMC to a neurturin solution does not increase the bioavailability of neurturin.

### Examples.

### Example 1.

### Plasma neurturin concentration following subcutaneous application of 0.05mg/kg neurturin formulated in 0.9% NaCl solution.

100 µl of a solution containing neurturin at concentration of 12.5 µg/ml formulated in physiological saline was subcutaneously injected into the neck region of mice. For each time point the average value of 3 plasma neurturin concentrations measured by an neurturin specific ELISA is plotted. Plasma concentrations of neurturin remain low, in the range of 1-2ng/ml. Compared to the total injected amount of neurturin, the bioavailbility is about 5%. The results are shown in Fig. 1.

### Example 2.

### Plasma neurturin concentration following subcutaneous application of 0.25mg/kg neurturin formulated in 0.9% NaCl solution alone or together with Heparin.

100 µl of a solution containing neurturin at concentration of 62.5 µg/ml formulated in physiological saline alone or together with heparin was subcutaneously injected into the neck region of mice. For testing the effect of the heparin formulation two different concentrations of heparin were added to the neurturin solution. For each time point the average value of 3 plasma neurturin concentration measured by an neurturin specific ELISA is plotted. Plasma concentrations of neurturin remain low following injection of neurturin in saline alone or formulated with 6.5 unit Heparin. Addition of 12.5 units to the injected neurturin significantly increased plasma concentration. Compared to the bioavailability of neurturin formulated in physiological saline the bioavailability of neurturin/12.5 U heparin formulation is about twofold increased. The results are shown in Fig. 2.

### Example 3.

### Plasma neurturin concentration following subcutaneous application of 0.25mg/kg neurturin formulated in 0.9% NaCl solution alone or together with Heparin or a low molecular weight heparin (LMWH).

100 µl of a solution containing neurturin at concentration of 62.5 µg/ml formulated in physiological saline alone or together with heparin or a LMWH (Fragmin) was subcutaneously injected into the neck region of mice. For testing the effect of the LMWH formulation two different concentrations of were added to the neurturin solution. For each time point the average value of 3 plasma neurturin concentrations measured by an neurturin specific ELISA is plotted. Plasma concentrations of neurturin remain low following injection of neurturin in saline alone. Addition of 6.5 units LMWH to the injected neurturin significantly increased plasma concentration. Furthermore, increasing the amount of LMWH in the formulation to 12.5 units increases neurturin plasma even further. Compared to the bioavailability of neurturin formulated in physiological saline the bioavailability of a neurturin formulation containing 12.5 units of a LMWH as an excipient is about tenfold increased. The results are shown in Fig. 3.

### Example 4.

### Plasma neurturin concentration following subcutaneous application of 0.05mg/kg neurturin formulated in 0.9% NaCl solution alone or together with a low molecular weight heparin (LMWH).

100 µl of a solution containing neurturin at concentration of 12.5 µg/ml formulated in physiological saline alone or together with a LMWH (Fragmin) was subcutaneously injected into the neck region. For testing the dose effect of the LMWH formulation four different concentrations of the LMWH were added to the neurturin solution. For each time point the average value of 3 plasma neurturin concentrations measured by an neurturin specific ELISA is plotted. Plasma concentrations of neurturin remain low following injection of neurturin in saline alone. Addition of a LMWH to the injected neurturin dose dependently increases plasma concentration of neurturin. The results are shown in Fig. 4.

### Example 5.

### Plasma neurturin concentration following subcutaneous application of 0.05mg/kg neurturin formulated in 0.9% NaCl solution alone or together with a pentosan polysulfat (PPS).

100 µl of a solution containing neurturin at concentration of 12.5 µg/ml formulated in physiological saline alone or together with PPS was subcutaneously injected into the neck region. For each time point the average value of 3 plasma neurturin concentrations measured by an neurturin specific ELISA is plotted. Plasma concentrations of neurturin remain low following injection of neurturin in saline alone. Addition of PPS to the injected neurturin increases plasma concentration. PPS compared to heparin or a LMWH increases neurturin plasma concentrations more efficiently when given at the same molar ratio. The results are shown in Fig. 5.

### Example 6.

### Plasma neurturin concentration following subcutaneous application of 0.05mg/kg neurturin formulated in 0.9% NaCl solution together with carboxymethylcellulose (CMC).

100 µl of a solution containing neurturin at concentration of 12.5 µg/ml formulated in physiological saline together with CMC was subcutaneously injected into the neck region. For testing the effect of the CMC formulation two different concentrations of were added to the neurturin solution. For each time point the average value of 3 plasma neurturin concentrations measured by an neurturin specific ELISA is plotted. Plasma concentrations of neurturin remain low following injection of a neurturin solution of saline together with CMC. Addition of CMC to a neurturin solution does not increase the bioavailability of neurturin. The results are shown in Fig. 6.

The following section of the description consists of numbered paragraphs simply providing statements of the invention already described herein. The numbered paragraphs in this section are not claims. The claims are set forth below in the later section headed "claims".
1. A pharmaceutical formulation comprising neurturin as an active ingredient and a low molecular weight polyanionic polymer having a weight average molecular weight (M_{w}) of up to about 12000 Da together with pharmaceutically acceptable carriers, diluents and/or adjuvants.
2. The formulation of statement 1, wherein neurturin is human neurturin or a pharmaceutically active fragment thereof.
3. The formulation of any of statements 1 to 2, wherein neurturin is linked to polyethyleneglycol.
4. The formulation of any of statements 1 to 3, wherein the weight average molecular weight (M_{w}) of the polyanionic polymer Is up to about 8000 Da.
5. The formulation of any one of statements 1 to 4, wherein the polyanionic polymer is a sulphate group-containing polymer.
6. The formulation of any one of statements 1 to 5, wherein the polyanionic polymer is selected from sulphated saccharides, sulphated cyclodextrins, sulphated acrylic polymers, and/or sulphated aromatic polymers, sulphated polyalcohols.
7. The formulation of statement 6, wherein the polyanionic polymer is selected from low molecular weight heparins or heparin derivatives, heparan sulphates, chondroitin sulphates, dextran sulphates, pentosan polysulphates or derivatives or combinations thereof.
8. The formulation of statement 7, wherein low molecular weight heparins and heparin derivatives are Enoxaparin, Dalteparin, Fragmin, Nadroparin, Tinzaparin, Fondaparinux, Bemiparin, Reviparin, Ardeparin, Certoparin, and/or Parnaparin, e.g. Lovenox^{®}, Fraxiparin^{®}, Sandoparin^{®} or Arixtra^{®}.
9. The formulation of statement 7, wherein the polyanionic polymer is a pentosan polysulphate with a weight-average molecular weight (M_{w}) of about 4000 to about 6000 Da.
10. The formulation of any one of statements 1 to 9, wherein the polyanionic polymer is a substantially non-anticoagulant polymer.
11. The formulation of any one of statements 1 to 10, wherein the polyanionic polymer is a substantially non-antiinflammatory polymer.
12. The formulation of any one of statements 1-11, which has an increased bioavailability of the active ingredient compared to a formulation without polymers.
13. The formulation of any one of statements 1 to 12, wherein the polyanionic polymer is present in an amount to provide an at least 2-fold, preferably at least 5-fold and more preferably at least 10-fold increase in the bioavailability of the active ingredient.
14. The formulation of any one of statements 1 to 13 for injection or infusion.
15. The formulation of any one of statements 1 to 14 for subcutaneous or intravenous injection.
16. Use of the formulation of any one of statements 1 to 15 for the preparation of a medicament for the prevention or treatment of pancreatic or neurodegenerative disorders.
17. Use of the formulation of any one of statements 1 to 15 for the preparation of a medicament for the prevention or treatment of diabetes mellitus type I, LADA and diabetes mellitus type II.
18. The formulation of any one of statements 1 to 15 for administration to a mammal, particularly a human,

## Claims

1. A pharmaceutical formulation comprising a heparin-binding protein growth factor as an active ingredient and a low molecular weight polyanionic polymer having a weight average molecular weight (M_{w}) of up to about 12000 Da together with pharmaceutically acceptable carriers, diluents and/or adjuvants.

2. The formulation of claim 1 wherein the growth factor is selected from neurturin, artemin, persephin, VEGF, bFGF an GDNF.

3. The formulation of claim 1 or claim 2, wherein the growth factor is a human growth factor.

4. The formulation of claim 2 or claim 3 wherein the growth factor is neurturin chemically derivatised with a water-soluble polymer.

5. The formulation of any of claims 2, 3 and 4 wherein the growth factor is neurtrin and the neutrin is for administration as a monotherapy or as a combination therapy, optionally together with a pharmaceutical agent selected from other pharmaceutical agents suitable for the treatment or prevention of pancreatic diseases and/or obesity and/or metabolic syndrome.

6. The formulation of claim 5 wherein said other pharmaceutical agents are selected from pharmaceutical agents suitable for stimulating and/or inducing differentiation of insulin producing cells from progenitor cells.

7. The formulation of any preceding claim wherein the weight average molecular weight (M_{w}) of the polyanionic polymer is up to about 8000 Da, and optionally is at least about 200 Da and more preferably at least about 500 Da.

8. The formulation of any preceding claim wherein the low molecular weight polyanionic polymer contains a plurality of sulphate groups.

9. The formulation of claim 8 wherein the polyanionic polymer is selected from low molecular weight sulphated saccharides, sulphated cyclodextrins, and sulphated synthetic polymers such as acrylic polymers, aromatic polymers, and/or polyalcohols.

10. The formulation of claim 9 wherein the polyanionic polymer is selected low molecular weight heparins or heparin derivatives, heparan sulphates, chondroitin sulfates, dextran sulphates, pentosan polysulphates or derivatives or combinations thereof.

11. The formulation of claim 10 wherein the polyanionic polymer is pentosan polysulfate with a molecular weight of about 4000 to about 6000 Dalton.

12. The formulation of claim 10 wherein the polyanionic polymer is selected from low molecular weight heparin (LMWH) analogues such as enoxaparin, dalteparin, fragmin, nadroparin, tinzaparin, fondaparinux, bemiparin, reviparin, ardeparin, certoparin, and/or parnaparin.

13. The formulation of any preceding claim which is for use in prevention and/or treatment of neurodegenerative or pancreatic disorders

14. The formulation of claim 13 which is for use in prevention and/or treatment of pancreatic autoimmune disorders, and optionally is for use in prevention and/or treatment of diabetes type I, LADA, or diabetes type II.

15. The formulation of any preceding claim which is for administration by injection or infusion.
